# EUROPEAN PATENT APPLICATION

(11) **EP 4 183 884 A1**
(43) Date of publication of application: **24.05.2023**
(21) Application number: 21209433.8
(22) Date of filing: 19.11.2021
(51) Int. Cl.: C12N 15/86

(54) **A METHOD OF PREPARING A FRACTION OF A STARTING POLYMER VECTOR FOR GENE THERAPY**

(71) Applicant: Branca Bunus Limited, 4 Dublin (IE)
(72) Inventor: Wang, Wenxin, Dublin, 4 (IE); Li, Yinghao, Dublin, 4 (IE); He, Zhonglei, Dublin, 4 (IE); A, Sigen, Dublin, 4 (IE); Tai, Hongyun, Dublin, 4 (IE)
(74) Representative: Purdylucey Intellectual Property

(57) **Abstract**

A method of preparing a fraction of a starting polymer comprising the steps of dissolving the starting polymer in a first solvent to provide a first polymer solution, providing a first precipitant comprising a mixture of the first solvent and a second solvent in a first volumetric ratio, wherein the solubility of the starting polymer in the second solvent is less that the solubility of the polymer in the first polymer, adding the first polymer solution to the first precipitant to provide a first mixed solution, allowing the first mixed solution to separate into a first precipitate and a first eluate; and, and separating the first precipitate from the eluate, wherein the first precipitate and/or the first eluate are fractions of the starting polymer having a molecular weight distribution that is narrower than the molecular weight distribution of the starting polymer. step of removing solvent from the first eluate. A composition comprising a nucleic acid component complexed together with a precipitate or an eluate obtained according to the method of the invention is also described, as is the use of the compositions in gene therapy.

## Description

### Field of the Invention

The present invention relates to a method of preparing a fraction of a starting polymer that has a narrower molecular weight distribution than the starting polymer. The invention also relates to fractions of the starting polymer obtained according to the method of the invention, and to the use of the fractions as nucleic acid delivery vehicles in therapeutic applications.

### Background to the Invention

Numerous medical diseases arise as a result of abnormalities to an individual's genetic code, leading to mutations that culminate in genetic disorders. Such genetic diseases account for considerable social and economic burdens globally and more importantly lead to severe lifelong negative impacts on the livelihood of suffering patients.

The ability to restore a protein's expression ("gene addition") or edit one's genetic code ("gene editing") to correct such disease-causing mutations has long been heralded as a transformative medical treatment with the potential to cure a multitude of genetic conditions. However, a substantial challenge to the broad spectrum uses of gene therapy as a first in line treatment strategy remains. The delivery of a gene therapy to a patient's cells is not possible on its own due to physicochemical barriers preventing entry to cells. Since the inactivated virus vectors in conventional delivery brings substantial safety concerns such as immunogenicity and insertional mutagenesis, the non-viral vectors are more promising candidate. In particular, the cationic polymer carrier has the advantages of stable structure, easy mass production, easy modification, etc., which represents the long-term future of gene therapy.

In most situation, a relatively high molecular weight effect is often required for polymer vectors to achieve high transfection efficiency. However, due to the limitation of the polymerization method, common polymer carriers such as Polyethyleneimine (PEI), Poly(β-amino esters) (PAE) and their derivatives tend to achieve a wide MWD while achieving high molecular weight. Meanwhile, researchers realize that molecular weight significantly affects the performance of polymer carriers, and the optimal interval varies in different systems (cargos, cells, and vector structures). To optimize the best situation, multiple synthesis is required to obtain products with different molecular weights, which is very costly and time consuming. Moreover, the wide MWD increase the complexity in polyplex, hindering the understanding of polymer molecular weight related transfection mechanism. At the same time, the wider MWD leads to unreliable reproducibility and unstable results. These problems significantly impede mechanism investigation and clinical applications.

To obtain polymer vectors with a narrow MWD, the conventional method is to use the SEC system for fractionation. But this can only be prepared in a small amount in the laboratory and cannot meet the requirements of large-scale manufactory. Due to the lack of easy-to-operate methods, until today, there are few studies on polymers vectors with narrow MWD, and the effect of MW on gene transfection activity and possible mechanism is still unclear.

It is an objective of the invention to overcome at least one of the above-referenced problems.

### Summary of the Invention

The objective is met by the provision of a method of fractional elution that fractionates a starting polymer into one or more fractions having a narrower molecular weight distribution than the starting polymer. The method employs a precipitant comprising a mixture of first and second solvent in a specific ratio. The first solvent is a good solvent for the starting polymer and the second solvent that is not as good a solvent for the polymer. The starting polymer is dissolved in the good solvent, and then the dissolved polymer is mixed with the precipitant slowly with mixing, and then allowed to settle to form a precipitate and an eluate. The precipitate is separated from the eluate and has a MWD narrower than the starting polymer. In addition, the eluate can be treated to remove solvent (e.g., by rotary evaporation) and the resultant polymer fraction often also has a narrower MWD that the starting polymer. This process can be repeated in an iterative fashion to form second and third generation precipitates, in which each step wish precipitation step employs a precipitant with a greater proportion of good solvent, resulting in a precipitate with a narrower MWD. Tests indicate that the fractions obtained by the method of the invention maintain stable chemical structures, with no side reactions (degradation or crosslinking) occurring during the fractionation process. In addition, when used as delivery vehicles for nucleic acids in cells, the polymer fractions of the invention demonstrate good biocompatibility and good transfection efficiency.

In a first aspect, the invention provides a method of preparing a fraction of a starting polymer comprising the steps of:
dissolving the starting polymer in a first solvent to provide a first polymer solution;
providing a first precipitant comprising a mixture of the first solvent and a second solvent in a first volumetric ratio, wherein the solubility of the starting polymer in the second solvent is less that the solubility of the polymer in the first polymer;
adding the first polymer solution to the first precipitant to provide a first mixed solution;
allowing the first mixed solution to separate into a first precipitate and a first eluate; and
separating the first precipitate from the eluate.

The first precipitate and the first eluate are fractions of the starting polymer. Both generally have a molecular weight distribution that is narrower than the molecular weight distribution of the starting polymer.

In any embodiment, the method includes a step of removing solvent from the first eluate.

In any embodiment, the method includes additional steps of
dissolving the first precipitate in the first solvent to provide a second polymer solution;
providing a second precipitant comprising a mixture of the first solvent and the second solvent in a second volumetric ratio in which the amount of the first solvent relative to the second solvent is increased compared to the first volumetric ratio;
adding the second polymer solution to the second precipitant to provide a second mixed solution;
allowing the second mixed solution to separate into a second precipitate and a second eluate; and
separating the second precipitate from the eluate.

In any embodiment, the method comprises a step of removing solvent from the second eluate.

In any embodiment, the method includes the additional steps of
dissolving the second precipitate in the first solvent to provide a third polymer solution;
providing a third precipitant comprising a mixture of the first solvent and the second solvent in a third volumetric ratio in which the amount of the first solvent relative to the second solvent is increased compared to the second volumetric ratio;
adding the third polymer solution to the second precipitant to provide a third mixed solution;
allowing the third mixed solution to separate into a third precipitate and a third eluate; and
separating the third precipitate from the third eluate.

In any embodiment, the method includes a step of removing solvent from the second eluate.

In any embodiment, the polymer solution is added to the precipitant dropwise.

In any embodiment, the polymer solution is added to the precipitant with stirring/agitation.

In any embodiment, solvent is removed from an eluate by rotary evaporation.

In any embodiment, the starting polymer is a Poly(β-amino esters) (PAE), in which the first solvent is acetone and the second polymer is optionally diethyl ether.

In any embodiment, the starting polymer is a cationic polymer. In any embodiment, the starting polymer is a polymer vector for gene therapy.

In any embodiment, the polymer is selected from a PEG-based polymer, Polyethyleneimine (PEI), Poly(β-amino esters) (PAE), poly(amido amine)s (PAA), poly(amidoamine) (PAMAM), poly-L-lysine (PLL), and their derivatives.

In any embodiment, the first precipitant has a volumetric ratio of 5:95 to 30:70.

In any embodiment, the method of the invention comprises 4, 5, 6, 7, 8 or 9 rounds of elution fractionation.

In another aspect, the invention provides a precipitate obtained according to a method of the invention. The precipitate may be a first precipitate, second precipitate, third precipitate, or n^{th} precipitate.

In another aspect, the invention provides an eluate obtained according to a method of the invention. The eluate may be a first eluate, second eluate, third eluate, or n^{th} eluate. Typically, the eluate has been treated to remove solvent, for example by rotary evaporation.

In any embodiment, the precipitate or eluate of the invention has a polydispersity index (PDI) of less than 4.0, 3.5, 3.0, 2.5, 2.4, 2.3, 2.2, 2.1 2.0 or 1.5.

In another aspect, the invention provides a pharmaceutical composition comprising a precipitate or eluate formed according to a method of the invention and a suitable pharmaceutical excipient.

In another aspect, the invention provides a composition comprises a nucleic acid component complexed together with polymer (i.e., a precipitate or eluate) of the invention or formed according to the invention. The composition is referred to herein as a "polyplex". The nucleic acid component may be a nucleic acid, a complex of a nucleic acid and a protein/peptide or combination of proteins or peptides (ribonucleoprotein). In one embodiment, the nucleic acid component is a nucleic acid, a gene editing system, a plasmid, a minicircle, a nanoplasmid, or an expression vector. The nucleic acid may be DNA, RNA or a combination of both. RNA may be employed in a format consisting of but not limited to, siRNA, dsRNA, shRNA, microRNA, crRNA, tracRNA, sgRNA, mRNA, RNA oligonucleotides, antisense oligonucleotides. The gene editing system may be a CRISPR-associated Cas system (Sander and Joung (2014) CRISPR-Cas systems for editing, regulating and targeting genomes Nature Biotechnology 32(4): 347-355)), a TALEN system (Boch J (February 2011); "TALEs of genome targeting". Nature Biotechnology. 29 (2): 135-6. doi:10.1038/nbt.1767. PMID 21301438), a meganuclease system, or a zinc finger nuclease (ZFN) system (Carroll, D (2011) "Genome engineering with zinc-finger nucleases". Genetics Society of America. 188 (4): 773 78doi:10.1534/genetics.111.131433. PMC 3176093. PMID 21828278). In one embodiment, the gene editing system is configured to perform insertational mutagenesis on a cell, for example OBLIGARE systems, and CRISPR-Cpf1 systems (Maresca et al. (2013) Obligate Ligation-Gated Recombination (ObLiGaRe): Custom-designed nuclease-mediated targeted integration through nonhomologous end joining Genome Res. 23: 539-546; see also WO2014/033644), Fagerlund et al. (2015) The Cpf1 CRISPR-Cas protein expands genome-editing tools Genome Biology 16: 251-253; Ledford (2015) Bacteria yield new gene cutter Smaller CRISPR enzyme should simplify genome editing Nature 526: 17).

The compositions of the invention may be employed generally in gene therapy, for example in gene addition, gene replacement, gene knockdown and gene editing. Gene replacement is defined as the provision of a functional healthy copy of a gene to replace a dysfunctional mutant containing gene which has given rise to a disease. Gene addition is defined as the supplementation of therapeutic genes that target a specific aspect of a disease mechanism. Gene knockdown is defined as the process of inhibiting a target genes capability to synthesize a toxic/dysfunctional protein which gives rise to a disease. Gene editing is defined as the process whereby a target genes nucleotide sequence is altered resulting in a loss of function/correction/manipulation of gene expression. Such gene editing systems consists of but are not limited to i) clustered, regularly interspaced, palindromic repeats (CRISPR)-associated (Cas) system; (ii) a transcription activator-like effector nuclease (TALEN) system; or (iii) a zinc finger nuclease (ZFN) system.

In one embodiment, the composition has a particulate form. In one embodiment, the particulate composition has a particle size of less than 2 µm, 1.5 µm, 1000 nm, for example 20-900 nm, 50-800 nm, 50-700 nm, 50-600 nm, 50-500 nm, 50-400 nm, 50-300 nm, 100-300 nm.

In a fourth aspect, the invention provides a polymer (i.e., a precipitate or eluate) of the invention or formed according to the invention for use as a nucleic acid delivery vehicle in for example a method of gene therapy.

Other aspects and preferred embodiments of the invention are defined and described in the other claims set out below.

### Brief Description of the Figures

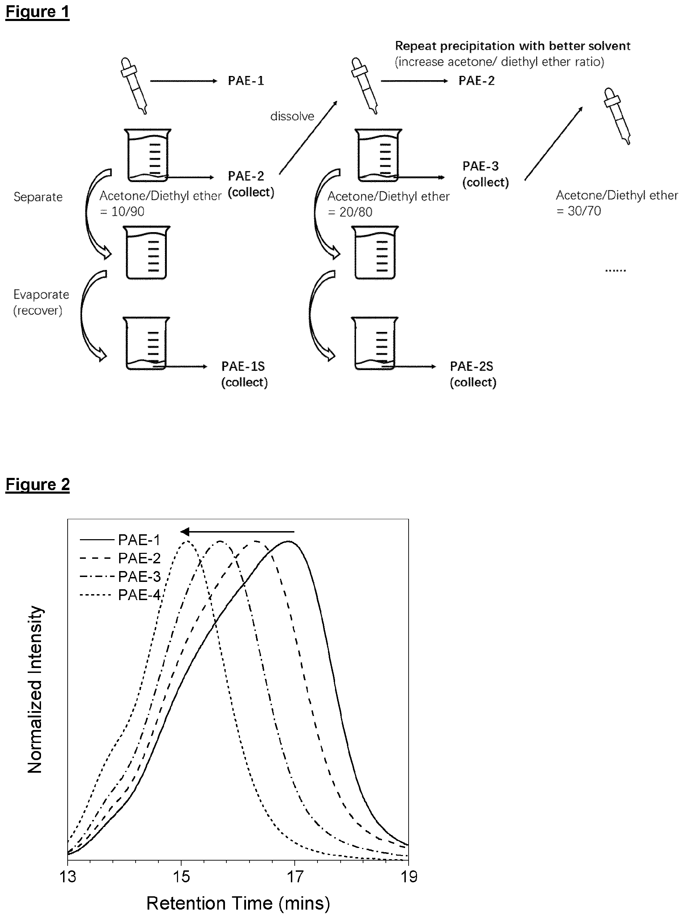

FIG. 1 is a diagram illustrating the method of the invention
**FIG.2**: GPC traces of starting polymer (PAE-1) and fractions of PAE-1 obtained via elution fractionation.
**FIG.3**: GPC traces of fractions of PAE-1 obtained via elution fractionation.
**FIG. 4****:** NMR spectrum of PAEs after elution fractionation. The results show that PAEs maintain stable chemical structures, no side reactions (degradation or crosslinking) happened during the fractionation process.
**FIG. 5****:** Illustration of the narrow molecular weight PAE obtained after fractional purification displayed good biocompatibility post 48 hours transfection. Only the PAE-4 group with highest molecular weight showed slight cytotoxicity.
**FIG. 6****:** Transfection efficiency of the polyplexes formed with PAE's.
**FIG. 7A** and **FIG. 7B****:** Transfection efficiency by fluorescence GFP signal in a fluorescence microscope.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g., a feature, element, characteristic, property, method/process step or limitation) or group of integers (e.g., features, element, characteristics, properties, method/process steps or limitations) but not the exclusion of any other integer or group of integers. Thus, as used herein the term "comprising" is inclusive or openended and does not exclude additional, unrecited integers or method/process steps.

"Starting polymer" refers to the polymer being treated according to the method of the invention. The starting polymer generally has a wide molecular weight distribution. The starting polymer may be a polymer that is commonly employed to deliver therapeutic payloads (such as nucleic acids) to cells. In one embodiment, the starting polymer is a cationic polymer. Examples of starting polymers include PEG-based polymers, Polyethyleneimine (PEI), Poly(β-amino esters) (PAE), poly(amido amine)s (PAA), (PAMAM), and poly-L-lysine (PLL), and their derivatives. Hyperbranched PAE's are described in WO2016/020474 and WO2021/058491. PAAs and PAMAM are described in EP2732821A1 and EP2388016A9. PLLs are described in EP1083882B1. PEG and PEI are described in CN103275329A The starting polymer may be a crude polymer. The starting polymer may be multiple topologies, including linear, branched, cyclic, star. Brush-liked polymers.. The starting polymer may have a polydispersity index of greater than 3.0, 3.5, 4.0, 4.5 or 5. The starting polymer may have a molecular weight of from 10 KDa to 20 KDa. The starting polymer may be a step-growth polymerization product, chain-growth polymerization product or ring-opening polymerization.

"First solvent" refers to a solvent that is a good solvent for the starting polymer, e.g. a solvent that can readily dissolve the starting polymer. Generally, the starting polymer can be dissolved by a small amount of the first solvent at a high concentration (>10mg/ml) at ambient pressure and 20°C. For PEA, acetone is a good first solvent.

"Second solvent" refers to a solvent that is not as good a solvent for the starting polymer compared with the first solvent (e.g., it is capable of dissolving less starting polymer compared with the first solvent at the same conditions). Generally, the solubility of the starting polymer in the second solvent is not more that 1mg/ml at ambient pressure and 20°C. For PEA, the second solvent may be diethyl ether.

An example of first and second solvents for a range of polymers is provided in Table 1 below:

**TABLE 1**

| **Polymer** | **First solvent** | **Second solvent** |
|---|---|---|
| PEA | Acetone | Diethyl ether |
| PEI | Water | Diethyl ether |
| PAA | Acetone | Diethyl ether |
| PAMAM | Methanol | Diethyl ether |
| PLL | Water | Diethyl ether |

"Precipitant" refers to a mixture of the first and second solvent in a defined volumetric ratio. The method may comprise one or more rounds of fractionation in which each round generally employs a precipitant (e.g., first precipitant, second precipitant, third precipitant, etc) that employs the same solvents but in a different volumetric ratio. The relative amount of the first solvent to the second solvent generally increases at each round of precipitation; for example, the volumetric ratio of the first and second solvents may be 10:90 in the first precipitant, 30:70 in the second precipitant, 50:50 in the third precipitant, etc. Generally, the first precipitant (e.g., the precipitant used in the first round of fractional elution) contains less than 50%, 40%, 30% or 20% by volume of the first solvent.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### EXAMPLE 1

The starting polymer **(PAE-1)** is a crude Poly (β-amino esters) (PAE-1). PAE-1 has a polydispersity index (PDI) of 3.1 (Table 2).

PAE-1 was dissolved in acetone at a concentration of 100mg/ml.

The first precipitant was prepared by mixing acetone as the first solvent with ethyl acetate as the second solvent in a volumetric ratio (first solvent: second solvent) of 10:90.

The polymer solution was then slowly added dropwise into the mixed precipitation agent with continuous stirring.

After the addition is complete, stop stirring and wait for the mixed solution to layer. Then collect the precipitate (**PAE-2**) As indicated in Table 2 below, the PDI of PAE-2 is 2.7, a lower PDI compared with the starting polymer demonstrating that PAE-2 has a narrower molecular weight distribution than PAE-1.

### EXAMPLE 2

The eluate from Example 1 was processed in a rotary evaporator to remove solvent and provide a polymer fraction PAE-1S. As indicated in Table 2 below, the PDI of PAE-1S is 2.2, a lower PDI compared with the starting polymer PAE-1 demonstrating that PAE-1S has a narrower molecular weight distribution than PAE-1.

### EXAMPLE 3

PAE-2 (the precipitate from Example 1) was dissolved in acetone at a concentration of 100mg/ml.

A second precipitant was prepared by mixing acetone as the first solvent with ethyl acetate as the second solvent in a volumetric ratio (first solvent: second solvent) of 20:80.

The PAE-2 solution was then slowly added dropwise into the second precipitant with continuous stirring.

After the addition is complete, stop stirring and wait for the mixed solution to layer. Then collect the precipitate (**PAE-3**) As indicated in Table 2 below, the PDI of PAE-3 is 2.4, a lower PDI compared with the PAE-2 demonstrating that PAE-3 has a narrower molecular weight distribution than PAE-2.

### EXAMPLE 4

The eluate from Example 3 was processed in a rotary evaporator to remove solvent and provide a polymer fraction PAE-2S. As indicated in Table 2 below, the PDI of PAE-2S is 2.1, a lower PDI compared with the starting polymer PAE-1 demonstrating that PAE-2S has a narrower molecular weight distribution than PAE-1 and PAE-1S

### EXAMPLE 5

PAE-3 (the precipitate from Example 3) was dissolved in acetone at a concentration of 100mg/ml.

A second precipitant was prepared by mixing acetone as the first solvent with ethyl acetate as the second solvent in a volumetric ratio (first solvent: second solvent) of 30:70

The PAE-3 solution was then slowly added dropwise into the second precipitant with continuous stirring.

After the addition is complete, stop stirring and wait for the mixed solution to layer. Then collect the precipitate (PAE-4) As indicated in Table 2 below, the PDI of PAE-4 is 2.0, a lower PDI compared with the PAE-3 demonstrating that PAE-4 has a narrower molecular weight distribution than PAE-2.

### EXAMPLE 6

The eluate from Example 5 was processed in a rotary evaporator to remove solvent and provide a polymer fraction PAE-3S. As indicated in Table 2 below, the PDI of PAE-3S is 1.7, a lower PDI compared with the starting polymer PAE-1 demonstrating that PAE-3S has a narrower molecular weight distribution than PAE-1 and PAE-2S

**TABLE 2**

| | **Mn** | **Mw** | **PDI** |
|---|---|---|---|
| **PAE-1** | 4859 | 14989 | 3.1 |
| **PAE-2** | 6493 | 17604 | 2.7 |
| **PAE-3** | 9893 | 23613 | 2.4 |
| **PAE-4** | 17440 | 34954 | 2.0 |
| **PAE-1S** | 3052 | 6893 | 2.2 |
| **PAE-2S** | 4887 | 10453 | 2.1 |
| **PAE-3S** | 6901 | 12085 | 1.7 |

### Equivalents

### EXAMPLE 7

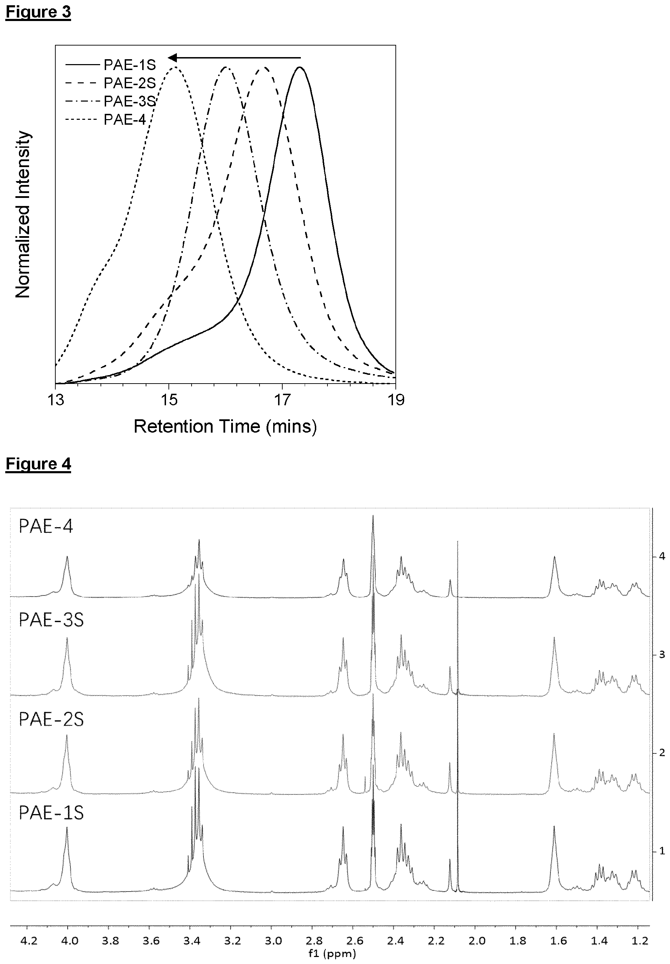

Proton Nuclear Magnetic Resonance (1H-NMR) Measurement:
The chemical structure of PAEs were confirmed by 1H-NMR. PAEs were dissolved in deuterated chloroform (CDCl3) and measurements were carried out on a 400 MHz Bruker NMR equipment. Figure 4 shows the NMR spectrum of PAEs after elution fractionation. The results show that PAEs maintain stable chemical structures, no side reactions (degradation or crosslinking) happened during the fractionation process.

### EXAMPLE 8

Molecular Weight Determination by Gel Permeation Chromatography (GPC): Molecular weight and polydispersity of the PAEs were measured by GPC. A 50 µL of the reaction mixture was collected and diluted into 1 mL DMF, filtered through a 0.22 µm filter and then analyzed using an Agilent 1260 GPC/SEC instrument equipped with a triple detector at 60 °C. DMF was used as the mobile phase and the flow rate was 1 mL per min. Poly(methyl methacrylate) was used as the standard sample for GPC calibration.

### EXAMPLE 9

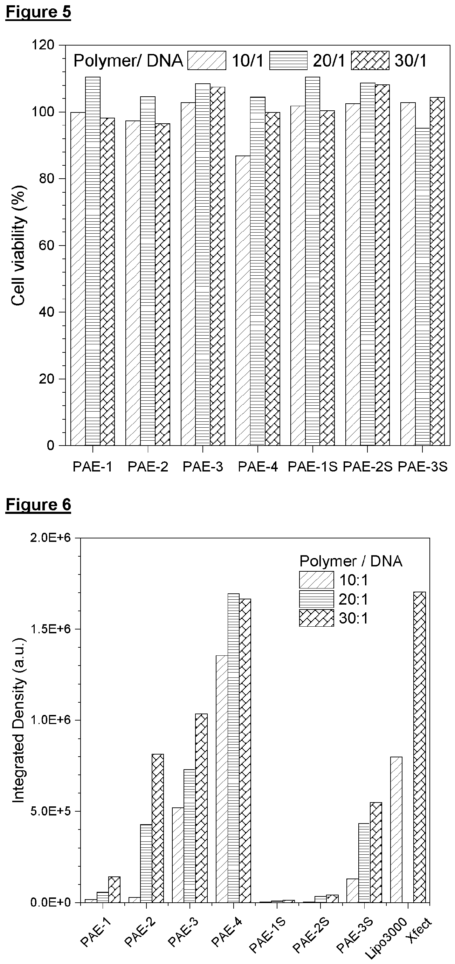

Figure 5 shows that the narrow molecular weight PAE obtained after fractional purification displayed good biocompatibility post 48 hours transfection. Only the PAE-4 group with highest molecular weight showed slight cytotoxicity. Cell viability of polyplexes was determined by the method of Alamarblue assay (Nano Lett. 19, 381-391 (2019)). The polyplexes were manufactured according to the method of previous publication (Sci Adv 2, e1600102 (2016)).

### EXAMPLE 10

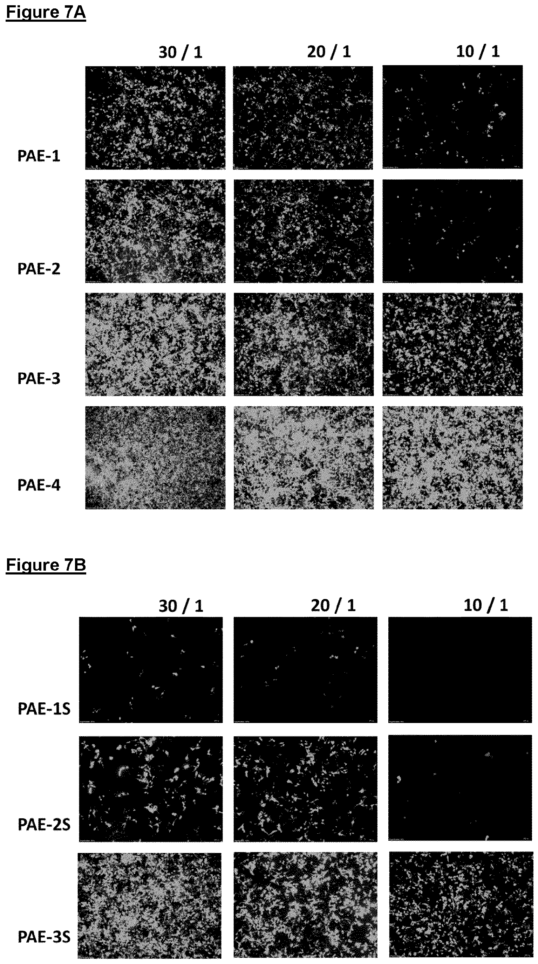

Figures 6 and 7 show the fluoresce emission of the GFP marker after transfection with a gWIZ plasmid by different fractionated polymer/DNA ratios in HEK cells. PAE-4, which has the narrowest MWD and the highest molecular weight after multistage precipitation, shows the highest transfection efficiency among PAE-1 to PAE-4. Compared with the crude polymer PAE-1, the expression of GFP increased nearly 100 times. At the same time, among those polymers with similar and narrow MWD (PAE-1S to PAE-4), the transfection efficiency increases as the molecular weight increases.

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method of preparing a fraction of a starting polymer comprising the steps of:
dissolving the starting polymer in a first solvent to provide a first polymer solution;
providing a first precipitant comprising a mixture of the first solvent and a second solvent in a first volumetric ratio, wherein the solubility of the starting polymer in the second solvent is less that the solubility of the polymer in the first polymer;
adding the first polymer solution to the first precipitant to provide a first mixed solution;
allowing the first mixed solution to separate into a first precipitate and a first eluate; and
separating the first precipitate from the eluate, wherein the first precipitate and/or the first eluate are fractions of the starting polymer having a molecular weight distribution that is narrower than the molecular weight distribution of the starting polymer.

2. A method according to Claim 1, including a step of removing solvent from the first eluate.

3. A method according to Claim 1 or 2, including the steps of
dissolving the first precipitate in the first solvent to provide a second polymer solution;
providing a second precipitant comprising a mixture of the first solvent and the second solvent in a second volumetric ratio in which the amount of the first solvent relative to the second solvent is increased compared to the first volumetric ratio;
adding the second polymer solution to the second precipitant to provide a second mixed solution;
allowing the second mixed solution to separate into a second precipitate and a second eluate; and
separating the second precipitate from the eluate.

4. A method according to Claim 2, including a step of removing solvent from the second eluate.

5. A method according to Claim 3 or 4, including the steps of
dissolving the second precipitate in the first solvent to provide a third polymer solution;
providing a third precipitant comprising a mixture of the first solvent and the second solvent in a third volumetric ratio in which the amount of the first solvent relative to the second solvent is increased compared to the second volumetric ratio;
adding the third polymer solution to the second precipitant to provide a third mixed solution;
allowing the third mixed solution to separate into a third precipitate and a third eluate; and
separating the third precipitate from the third eluate.

6. A method according to Claim 2, including a step of removing solvent from the second eluate.

7. A method according to any preceding Claim, in which the polymer solution is added to the precipitant dropwise with stirring/agitation.

8. A method according to any preceding Claim, in which solvent is removed from the eluate by rotary evaporation.

9. A method according to any preceding Claim, in which the starting polymer is a cationic polymer.

10. A method according to any preceding Claim, in which the starting polymer is a Poly(β-amino esters) (PAE), in which the first solvent is acetone and the second polymer is ethyl acetate, and in which the first precipitant optionally has a volumetric ratio of 5:95 to 30:70.

11. A first precipitate obtained according to a method of Claim 1.

12. A second precipitate obtained according to a method Claim 3.

13. A first eluate obtained according to a method of Claim 2.

14. A second eluate obtained according to a method Claim 4.

15. A composition comprising a nucleic acid component complexed together with precipitate of Claim 11 or 12 or an eluate of Claim 14 or 15.
